# EUROPEAN PATENT SPECIFICATION

(11) **EP 0 626 455 B1**
(45) Date of publication and mention of the grant of the patent: **15.07.1998**
(21) Application number: 93830238.7
(22) Date of filing: 28.05.1993
(51) Int. Cl.: C12Q 1/24, C12N 11/04

(54) **Method of analysis, separation and micro-encapsulation of cell elements**
Verfahren zur Analyse, Abtrennung und Mikroverkapselung von zellulären Elementen
Méthode d'analyse, de séparation et de microencapsulation d'éléments cellulaires

(43) Date of publication of application: 30.11.1994
(73) Proprietor: ENEA ENTE PER LE NUOVE TECNOLOGIE, L'ENERGIA E L'AMBIENTE, I-00198 Roma (IT); BECTON DICKINSON S.p.A., I-20153 Milano (IT)
(72) Inventor: Lucretti, Sergio, I-00060 Rome (IT)
(74) Representative: de Simone, Domenico

(56) References cited:
- EP-A- 0 022 434
- P.J. DIX (EDITOR) 'Plant Cell Line Selection Procedures and Applications' 1990 , VCH PRESS , WEINHEIM, DE * page 39 - page 50 * ANDREW LISTER: " Flow cytometry for Selection of Plant Cells in vitro"
- BIOTECHNOLOGY AND BIOENGINEERING SYMPOSIUM vol. 17 , 1986 , NEW YORK, NY, US pages 185 - 195 JAMES C. WEAVER 'Gel Microdroplets for Microbial Measurement and Screening'
- BIOTECHNOLOGY vol. 9, no. 9 , September 1991 , NEW YORK US pages 873 - 877 JAMES C. WEAVER ET AL. 'Rapid Clonal Growth Measurements at the Single-cell Level: Gel Microdroplets and Flow Cytometry'
- METHODS IN CELL BIOLOGY vol. 26 , 1982 , NEW YORK, NY, US pages 278 - 295 DAVID R. PARKS AND LEONARD A. HERZBERG 'Fetal Cells from Maternal Blood: Their Selection and Prospects for Use in Prenatal Diagnosis'
- BIOTECHNOLOGY vol. 3, no. 9 , September 1985 , NEW YORK US pages 811 - 816 CLAUDIO L. ALFONSO ET AL. 'Selection of somatic Hybrid Plants in Nicotiana through Fluorescence-activated Sorting of Protoplasts'
- JAPANESE JOURNAL OF BREEDING vol. 33, no. 2 , February 1983 , TOKYO pages 119 - 122 SHOZO KOBAYASHI ET AL. 'Plant Regeneration from 'Trovita' Orange Protoplasts'
- S. MANTELL AND H. SMITH (EDITORS) 'Plant Biotechnology' 1986 , CAMBRIDGE UNIVERSITY PRESS , CAMBRIDGE, GB K. LINDSEY and M.M. YEOMAN: ' Novel experimental systems for studiying the production of secondary metabolites by plant tissue cultures' * page 39 - page 66 *

## Description

The invention relates to a method to select cell elements, including mono-, multi- or sub-cell elements, that is specially useful for vegetal cell treatments when flow-cytometry procedures are involved.

The modern biotechnologies made practical to manipulate the genetic properties of organisms, embryos and cells by stable introduction of exogeneous genes, that provide a new phenotype to recipient cells, such as resistance against patogenic agents or environmental stress, or capability to generate new metabolites. Also somatic hybridation or high-level of secondary metabolite induction techniques (2) are comprised in the cell manipulation methods.

When vegetal cells are involved, due to the totipotency thereof, regenerate plants could be obtained having new characteristics, which could be very largely and advantegeously used in the agronomy, industry and food production fields (3).

A main limiting reason in spreading the cell manipulation methods to a large extent is the problem to detect and select the manipulated cells, as often they result as a very low fraction of the whole cell population. In order to overcame such problem, cell lines are used as recipient cells, that are selected according to special requirements, such as the ability to be cultivated in the presence of selective toxic substances. However, the use of such "laboratory" cell lines is a further limit to apply these methods on agro-industrial scale.

The detection methods of manipulated cells by fluorescent markers, vital dyes, molecular probes or antibodies, associated to the flow-cytofluorometry, allow virtually to use any type of cell or cell sub-structure through a qualitative and quantitative analysis of the fluorescent emission and the automatic quick separation of the interesting elements. The cell elements, having a fluid substrate as vehicle, are intercepted once at the time, and the signal quality and intensity analyzed. The corresponding signal to any single cell element could be identified and separated from the other element signals by means of an electrical polarization and micro-drop fractionating mechanism of the cell flow, that is controlled by the vibrations of a piezoelectric crystal. The electrical charged droplets, containing the actual interesting elements, could be separated through deviating its from the normal trajectories when passing through a stationary electric field, and then collected under sterilization conditions into any suitable container.

A number of examples of these techniques are found in animal and microbiology fields, while very limited uses concerned the vegetal biotechnology (1). This is mainly due to the brittleness features as well as to sizes of the vegetal cells, that poorly meet the requirements of the normal flowcytofluorometry instruments for animal cells, yet susceptible to move into a fluid (such as, for inst. within the blood). On the contrary, the vegetal cells are obtained from the plant tissues through the enzymic hydrolysis of the cellulose interconnecting structures, without which they lack rigidity and mechanical strength. Moreover, due to the presence of a large vacuole in the vegetal cell cytoplasm; which represents appr. the 90 percent of the total volume and implies an appr. 1000 time larger volume than the animal cell volume, a high cell breakage fraction occurs during the isolation procedure, and accordingly a limited recovery of the interesting cells usually results.

The inventor overcame the problems of the existing art by providing a method to analyze, sort and enencapsulate selected cell elements, such as single cells, sub-cell structures or multi-cell aggregates, from an inert substrate, which allows to isolate the cell elements as above at high efficiency level. The use of such methods made very more effective the cell manipulation technology, associated with the flow-type cytofluoro- metry, specially when vegetal cells are involved.

The cell elements are resuspended in a fluid medium, comprising an inert material, having the ability to solidify under certain conditions.

Surprisingly this fluid medium showed an ability to be used in a flow-cytofluorometry procedure, through which the particles could be both qualitatively and quantitatively analyzed, then separated as microdroplets using a fractionation procedure; each droplet containing only one element, and, under proper conditions, solidifying instantaneously, though maintaining the integrity of each involved element.

The collected material is purified at a high level (90-99 percent of purity) and only contains the preselected elements through the cytoflurometry analysis, and therefore could be available for subsequent in vitro manipulations in order to obtain:
- multicell structures (in the case of vegetal cells similar to the artificial seeds), surely generated from a single cell (cloning);
- an isolation of subpopulation cells as well as single cells (clones) having a high metabolic activity and an ability to bio-chemically convert special substrates and/or to yield secondary metabolism products with high added value.

Moreover, the ability of single cells to be manipulated within a solidified droplet (that could be defined as chemical "microreactors") considerably facilitates the recovery of excreta from the culture medium or the biochemically converted material from the same cells.
The process according to this invention allows to use more effectively the flow-type cytofluorometry as an instrument to analyze and separate vegetal cell as well as cell elements, by optimizing the recovery of unbroken structures into an inert matrix, which is perfectly compatible with, and advantageous for the cell growth. This provides a clear improvement in developing methods of in vitro selection of vegetal elements from high agronomic value organisms by preventing the need of the availability of any laboratory strain or previously manipulated material, to which suitable characters were added in order to make the selection practicable (such, for inst., strength against antibiotics, or antimetabolites, etc.).

The separated cells and the cell elements using the method of this invention maintain perfectly its integrity and are collected in a considerably higher percentage than the existing art methods, such as the unmodified analysis and separation procedure, and also they are able to be manipulated and/or to grow until the desired final product is obtained.

Therefore this invention allows:
- to optimize the isolation and the recovery of animal or vegetal intact cells (shock protection by the solidified material);
- to obtain multi-cell clones and organized embrionic structures (artificial seeds, only as far as the vegetal cells are concerned);
- to provide "cell microreactors" that are able to generate and/or to convert active secondary metabolites;
- to isolate, grew and recover microorganism colonies from a single microorganism;
- to encapsulate nuclei, Altmann's granules, chloroplasts and chromosomes for further cell manipulations and/or genetic conversions as well as for studies over its growth regulation and in vitro duplication mechanisms.

Therefore a scope of this invention is a method to analyze, separate and micro-encapsulate cell, sub cell or multicell elements, comprising to resuspend said elements in a fluid medium, at least comprising an inert substance, that is able to solidify in the presence of a catalyzer, being said catalyzer not comprised into said fluid medium; to feed said fluid medium to a container of the fluid to be supplied to a flow-type cytometer; this cytometer analysis comprising to fractionate said fluid medium in form of micro-sized droplets so that any droplet contains no more than only one of said elements; and to separate and collect said micro-sized droplets into containers wherein said solidification catalyzer is present.

According to a preferred embodiment of this invention, said method to analyze, separate and micro-encapsulate cell elements comprises a treatment of said elements with fluorochromes before said resuspension procedure and said separation occurs following a fluore- scent emission from said elements.

In an alternative embodiment according to this invention, said separation occurs by diffraction.

According to a preferred embodiment of this invention, said fluid medium loading to the container of the flow-type cytometer fed fluid is carried out after having filtered the fluid medium.

According to an embodiment of this invention said fluid medium comprises an isotonic salt solution and said inert solidifying material is selected from the product range as follows: alginate salts, agarose, agar-agar, carrageenan, gelatin, polyacrilamide, polystirene, dextran and precipitated metal hydroxides. Preferably, said solidifying material comprises sodium alginate and said catalyzer comprises a compound, which releases bi- or trivalent ions when in solution. More preferably said sodium alginate solution is at a concentration in the range from 0.2 and 1.0 percent and said catalyzer comprises calcium chloride at a concentration range from 10 to 100 mM. Most preferably, the sodium alginate con- centration is 0.5 percent and the calcium chloride concentration is 50 mM.

According to a preferred embodiment of this invention the cell elements have vegetal origin, and preferably are isolated from one of the following species: *Nicotiana benthamiana, Solanum tuberosum and Citrus sinensis.*

This invention will be described in the following by some explanatory, but not limiting examples with reference to the annexed figures, wherein:
fig. 1 is a schematic view of the flow-type cytofluoro metry system, the micro-droplet formation and the micro encapsulating procedure; and
fig.2 shows a diagram of micro-capsule dimension variation versus the analysis nozzle diameter.

### EXAMPLE 1

In order to allow a germ free collection of the interesting elements, the cytofluorometer (Facstar Plus Becton Dickinson, San José, California USA) section, wherein a fluid must circulate, was properly and repeatedly washed out with a 70 percent EtOH solution; it is to be outlined that a high capacity, on-line absolute filter in the circuit to sterilize the sheat fluid was maintained in contact with EtOH for at least 1 hour.

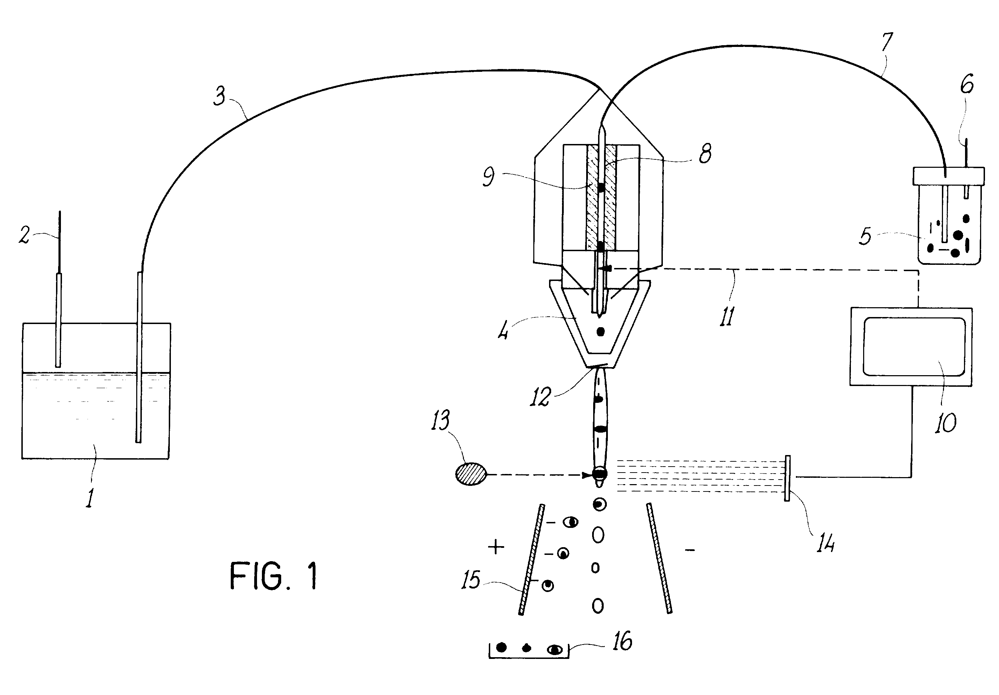

Figure 1 shows a scheme of the process: the fluid medium 1, containing the inert solidifying substance for the micro-encapsulation, is delivered by means of the pressurized air 2 through the line 3 to the chamber of focusing 4. At the same time, the particles 5 to be analyzed and separated are delivered by means of the pressurized air 6 through the line 7 to the chamber of focusing 4. The particles 5 are injected into the focusing chamber 4 by an injector 8 controlled by a pizoelectric crystal 9 for the controlled production of micro-drops. From a computer 10 a polarizing impulse 11arrives to the focusing chamber 4; the particles go through a nozzle 12 and a laser signal 13. The generated signal 14 goes back to the computer 10. The polarized particles are deviated by plates 15 with a costant voltage of 2500 V and are seeded into the recipient 16 containing the solidification catalyst.

The used cell elements were isolated from plants belonging to the following species: *Solanum tuberosum* and *Citrus sinensis*; however, other species could be successfully used.

Particularly, the *Citrus sinensis* protoplasts were isolated from "Tarocco" cultivar cell suspensions, which were obtained from nucellus callus, as described in (4).

The *Nicotiana benthamiana* protoplasts were isolated from leaves of *in vitro* cultivated seedings, 20-30 day aged, according to the procedure described in (5).

The isolated protoplasts were dipped into 1 µg/ml of FDA sigma for minutes at 25°C. Following the acetate hydrolysis from cytoplasm esterases, free fluorescein was generated, which emitted a green-yellow fluorescence when excited by a blue ligth; this substance had a tendency to concentrate in the cell interior.

The cell elements were placed into an isotonic culture medium, lacking of calcium and magnesium ions, in order to safeguard its structure and/or vitality and loaded in the cytofluorometric analysis vessels according to known methods to anyone skilled in the art.

In order to allow the encapsulating process, a 0.5 percent sodium alginate solution was prepared in a salt medium, free from calcium ions. This solution was loaded into the cytofluorometer feeding vessel and was the actual matrix, from which the droplets containing the cell elements were obtained.

The essential parameters to separate cell and sub-cell elements are summarized in the table 1 (see p.12).

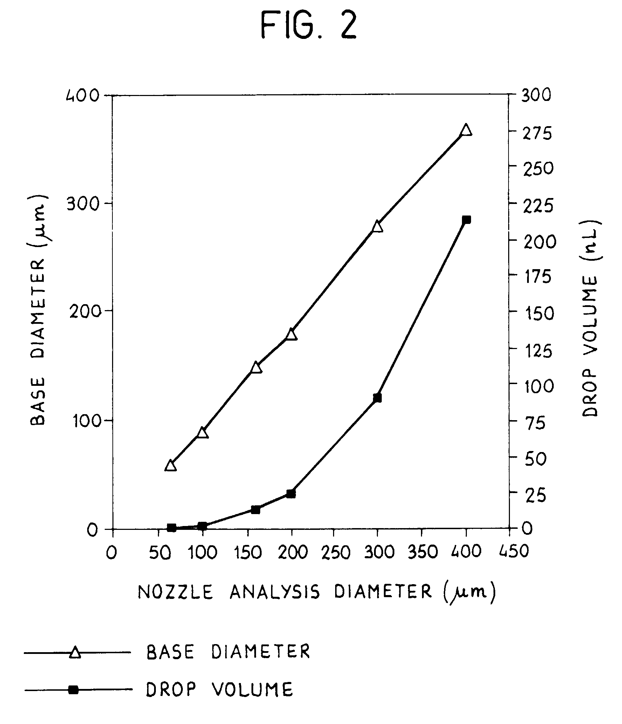

The analysis nozzle was selected as adequate to the particles to be selected and separated (usually, the nozzle diameter must be three or four times larger than the particle diameter, by taking into account the need to maintain the fluid flow rate at a minimum, in order to guarantee a higher analysis stability, without negatively affecting a correct sample hydrodynamic focusing for a correct element-by-element analysis according to a single-cell based technique (fig.2).

**Table 1**

| Essential Parameters To Separate Cell And Sub-Cell Elements | | | | | |
|---|---|---|---|---|---|
| Particles (larger size, µm) | Nozzle diameter analysis (µm) | Sheat fluid composition | Sheat fluid pressure (psi) | Vibration frequency (kHz) | Amplitude frequency (V) |
| chromosomes (2-10) | 65 (60-100) | KCl 80mM NaCl 20mM Na alginate 0.5% (w/v) pH 7.2 | 19.5 (21-15) | 28 (30-12) | 0.5 (0.5-2.0) |
| | | | | | |
| protoplasts (30-200) | 200 (100-500) | KCl NaCl H₂PO₄ Mannitol 0.3M Na alginate 0.5% (w/v) | 4 (6-2.5) | 6.1 (2-12) | 2 (0.5-25) |

The test variability ranges are reported in brackets.

Furthermore the sheat fluid flow was stabilized by removing any existing liquid and air bubble into the circuit by introducing EtOH at 70 percent into the focusing chamber.

Then the vibration piezoelectric source as well as the deflection plates, that allow to stabilize the temperature, were energized; the flow breaking point and the λ average interval among the droplets were established; the correction factor to be applied between the time of the fluorescent emission of any object and the corresponding drop delay was calculated and input to the apparatus to synchronize the polarization impulse on the drop containing the element, separation of which was desired. Normally the correction factor ranged from 6.0 and 9.0. The signal values of the interesting particles were defined and used as sorting gates.

A drop collecting vessel, containing a culture medium and 50 mM of CaCl₂ as solidification catalyzer was fitted.

The separation procedure was then started, by checking periodically that the flow rate was stable and the breaking point unchanged in order to detect immedia- tely any air bubble or on-line absolute filter obstruction occurrence. The summarizing data are shown in the table 2.

**Table 2**

| Summary of the cell parameters in the micro-encapsulation procedure | | | | | | |
|---|---|---|---|---|---|---|
| | Particle starting concentr. (part/ml) | Detecting parameter | Sorting efficiency (%) | Plating efficiency (%) | Separated Cell Nr. p.Vessel | Collect. Substr. |
| Chromosomes | 10⁶ | Dna (propidium iodide, 50 ug/ml) | 99 | = | 10⁴-10⁵ | *LB + CaCl₂ 50 mM |
| Protoplasts | 10⁴-10⁵ | Viabiity (FDA 1 ug/ml) | 95 (90-95) | Encapsul. | 1-10⁵ | Culture medium CaCl₂ 50 mM |
| | | | | A=90-60 | | |
| | | | | N=60-40 | | |
| | | | | Not encapsul. | | |
| | | | | A=50-30 | | |
| | | | | N=30-0 | | |

| | | | | | | |
|---|---|---|---|---|---|---|
| *LB = Lysis Buffer: chromosome extraction solution (ref.4); Encapsul. : micro-encapsulation in alginate; A = Orange tree; N = Nicotiana; Not encapsul. = alginate protection free. | | | | | | |

No toxic or disruptive effects were detected in the micro-encapsulating process due to the analyzed cell elements.

### EXAMPLE 2: Chromosome separation

The chromosome suspension were obtained from *Vicia faba*apical roots, after chemical synchronisation, formaldehyde fixation and mechanical grinding, as described in (2), but with less modifications.

The isolated chromosomes were dyed with 50 µg/ml propidium iodide (PI), that associated with the double strand nucleic acids and increased 10 times its orange- red fluorescence when excited by blue ligth. The PI-coloured chromosomes were excited with 200 mW at 514 nm. The fluorescence signals were collected through photomultipliers equipped with specific pass band filters (535 nm for fluorescein and 630 nm for propidium).

The concerned parameters are summarized in the previous tables 1 and 2.

### REFERENCES

(1) Weaver J.C., Bliss J.G., Powell K.T, Harrison G.I. and Williams G.B., "Rapid clonal growth measurements at the single-cell level: gel, microdroplets and flow cytometry", BIO/TECHNOLOGY, 9, 873-77, 1991.
(2) Lindsey K. and Yeoman M.M., "Novel experimental systems for studying the production of secondary metabolites by plant tissue culture" from " Plant Biotechnology", edited by Mantell S.H. and Smith H., Cambridge Univ. press, 39-66, 1986.
(3) Lister A., "G4Flowcitometry for selection of plant cells in vitro" from "Plant cell line selection procedures and applications", edited by Dix P.J., VCH press, 39-50, 1990.
(4) S. Kobayashi, H. Ucimaja and 1. Ikeda, "Plant regeneration from "Trovita" orange protoplasts", Jpn. J.Breeding, 33, 119, 1983.
(5) Alfonso C.L., Harkins K.R., Thomas Compton M.A., Krejci A. E. and galbraith D.W., " Selection of somatic hybrid plants in Nicotiana through fluorescence activated sorting of protoplasts", Bio/Technology, 3, 811-816, 1985.

## Claims

1. A method of analysis, separation and micro encapsulation of cell, sub-cell or multi-cell elements characterized in comprising the steps as follows:
- to resuspend said elements in a fluid medium, comprising at least an inert material that is able to solidify in presence of a catalyzer; being said catalyzer not comprised in said fluid medium;
- to load said fluid medium into a fluid feeding vessel of a flow-type cytometry apparatus;
- to carry out a cytometry analysis, comprising to fractionate said fluid in microdroplets, each one of which contains no more than only one element;
- to separate and collect said microdroplets within vessels containing said solidification catalyzer.

2. A method of analysis, separation and microencapsulation of cell, sub-cell or multi-cell elements according to the claim 1, wherein said resuspension follows a said element treatment with fluorochromes and said separation results from a fluorescence emission of said elements.

3. A method of analysis, separation and microencapsulation of cell, sub-cell or multi-cell elements according to the claim 1, wherein said separation results from a diffraction.

4. A method of analysis, separation and microencapsulation of cell, sub-cell or multi-cell elements according to any previous claim, wherein said loading step follows a filtering operation.

5. A method of analysis, separation and microencapsulation of cell, sub-cell or multi-cell elements according to any previous claim, wherein said fluid medium comprises an isotonic salt solution, and said inert solidifying material is selected from the following range: alginate salts, agarose, agar-agar, carrageenan, gelatin, polyacrilamide, polystirene, dextran and precipitated metal hydroxides.

6. A method of analysis, separation and microencapsulation of cell, sub-cell and multi-cell elements according to the claim 5, wherein said inert solidifying material comprises sodium alginate, and said catalyzer comprises a compound releasing bivalent or trivalent ions when solubilized.

7. A method of analysis, separation and microencapsulation of cell, sub-cell or multi-cell elements according to the claim 6, wherein said sodium alginate solution is at concentration ranging from 0.2 to 1.0 percent, and said catalyzing compound comprises calcium chloride at a concentration ranging from 10 to 100 mM.

8. A method of analysis, separation and microencapsulation of cell, sub-cell or multi-cell elements according to the claim 7, wherein the said sodium alginate concentration is 0.5 percent and said calcium chloride concentration is 50 mM.

9. A method of analysis, separation and microencapsulation of cell, sub-cell or multi-cell elements according to any previous claim, wherein said elements are from a vegetal source.

10. A method of analysis, separation and microencapsulation of cell, sub-cell or multi-cell elements according to the claim 9, wherein said vegetal elements are isolated cells from one of the following species: *Nicotiana benthamiana, Solanum tuberosum, Citrus sinensis,* or sub-cell elements thereof, including chromosome.

## Patentansprüche

1. Verfahren zur Analyse, Abtrennung und Mikroverkapselung von zelligen, subzelligen und vielzelligen Elementen, gekennzeichnet durch die folgenden Verfahrensschritte:
- Wiederaufschwemmen der vorgenannten Elemente in einem flüssigen Mittel, das mindestens einen, in Gegenwert eines Katalysator erstarrbaren Zuschlagstoff enthält, wobei der vorgenannte Katalysator im genannten flüssigen Mittel nicht enthalten ist;
- Einbringen des vorgenannte flüssigen Mittels in einen Flüssigkeit speisenden Behälter eines Durchfluss-Zytometers;
- zytometrische Analyse mit der Fragmentation der vorgenannten Flüssigkeit in Mikrotropfen, von denen jeder nicht mehr als ein Element enthält;
- Abscheidung und Aufnahme der genannten Mikrotropfen in Aufnahmemitel, die den vorgenannten Erstarrungskatalysator enthalten.

2. Verfahren zur Analyse, Abtrennung und Mikroverkapselung von zelligen, subzelligen und vielzelligen Elementen, nach Anspruch 1, dadurch gekenzeichnet, dass das vorgenannte Wiederaufschwemmen nach der Behandlung der vorgenanten Elemente mit Fluorchromen vorgenomen wird und die vorgenannte Abscheidung auf Grund der Fluoreszenz-Emission der vorgenannten Elemente stattfindet.

3. Verfahren zur Analyse, Abtrennung und Mikroverkapselung von zelligen, subzelligen und vielzelligen Elementen, nach Anspruch 1, worin die vorgenannte Abscheidung durch Diffraktion stattfindet.

4. Verfahren zur Analyse, Abtrennung und Mikroverkapselung von zelligen, subzelligen und vielzelligen Elementen, nach je einem der vorhergehenden Ansprüche, worin des vorgenannte Einbringen nach einer Filtration stattfindet.

5. Verfahren zur Analyse, Abtrennung und Mikroverkapselung von zelligen, subzelligen und vielzelligen Elementen, nach je einem der vorhergehenden Ansprüche, worin dass vorgenannte flüssige Mittel eine isotonische Salzlösung enthält und der vorgenannte erstarrungsfähige Zusehlagstoff aus der folgenden Gruppe ausgewählt ist: Alginatsalze, Agarose, Agar-Agar, Carrageen, Gelatin, Polyacrilamid, Polystirene, Dextran und niedergeschlagene Metallhydroxide.

6. Verfahren zur Analyse, Abtrennung und Mikroverkapselung von zelligen, subzelligen und vielzelligen Elementen, nach Anspruch 6, worin der vorgenannte erstarrbare Zusatzstoff Natriumalginate enthält und der vorgenannte Katalysator eine Verbindung enthält, die in Lösung zweiwertige und dreiwertige lone bildet.

7. Verfahren zur Analyse, Abtrennung und Mikroverkapselung von zelligen, subzelligen und vielzelligen Elementen, nach Anspruch 6, worin die genannte Natriumalginate-Lösung eine Konzentration zwischen 0,2% und 1,0% hat und die Katalysator-Verbindung eine Konzentration von Calcumchlorid zwischen 10 un 100 mM aufweist.

8. Verfahren zur Analyse, Abtrennung und Mikroverkapselung von zelligen, subzelligen und vielzelligen Elementen, nach je einem der vorhergehenden Ansprüche, worin die die Konzentration von NatriumAlginate 0,5% beträgt und die Konzentration von Calciumchlorid 50 mM ist.

9. Verfahren zur Analyse, Abtrennung und Mikroverkapselung von zelligen, subzelligen und vielzelligen Elementen, nach je einem der vorhergehenden Ansprüche, worin die vorgenannten Elemente von Pflanzen abstammen.

10. Verfahren zur Analyse, Abtrennung und Mikroverkapselung von zelligen, subzelligen und vielzelligen Elementen, nach Anspruch 9, worin die Pflanzen-Elemente aus einer der folgenden Species isolierte Zellen: Nicotiana-Behthamiana, Solanum Tuberosum, Citrus Sinensis oder Subzellen derselben, einschliesslich Chromosome sind.

## Revendications

1. Méthode d'analyse, de séparation et de microencapsulation d'éléments cellulaires, subcellulaires ou multicellulaires, caractérisé en ce qu'il comprend les phases suivantes:
- résuspension desdits éléments dans un moyen fluide, comprenant au moins une matière inerte susceptible de solidifier en presence d'un catalyseur de solidification, ledit catalyseur n'étant pas contenu dans ledit moyen fluide
- chargement dudit moyen fluide dans un récipient d'alimentation de fluid d'un cytomètre à flux;
- analyse cytomètrique comprenant la fragmentation dudit fluide en microgouttes, chacun ne contenant pas plus d'un seul élément:
- séparation et collectage desdites microgouttes dans les récipients contenant ledit catalyseur de solidification.

2. Méthode d'analyse, de séparation et de microencapsulation d'éléments cellulaires, subcellulaires ou multicellulaires, selon la revendication 1, dans lequel ladite résuspension a lieu après le traitement desdits éléments par fluorchromes et ladite séparation se passe par l'emission fluorescente desdit éléments.

3. Méthode d'analyse, de séparation et de microencapsulation d'éléments cellulaires, subcellulaires ou multicellulaires, selon la revendication 1, dans lequel ladit séparation est due à la diffraction.

4. Méthode d'analyse, de séparation et de microencapsulation d'éléments cellulaires, subcellulaires ou multicellulaires, selon une quelconque des revendications précédentes, dans lequel ladite phase de chargement a lieu après une opération de filtration.

5. Méthode d'analyse, de séparation et de microencapsulation d'éléments cellulaires, subcellulaires ou multicellulaires, selon une quelconque des revendications précédentes, dans lequel ledit moyen fluide comprend une solution saline isotonique et ladite matière inerte de solidification est choisie d'un groupe suivant: sels d'alginate, agarose, agar-agar, carageen, gelatine, polyacrilamide, polystirene, dextran et hydroxides métalliques précipités.

6. Méthode d'analyse, de séparation et de microencapsulation d'éléments cellulaires, subcellulaires ou multicellulaires, selon la revendication 5, dans lequel ladite matière inerte de solidification contien alginat de sodium et ledit catalyseur contien un composé qui produit en solution d'ions bivalents et trivalents.

7. Méthode d'analyse, de séparation et de microencapsulation d'éléments cellulaires, subcellulaires ou multicellulaires, selon la revendication 6, dans lequel ladite solution d'alginate de sodium a une concentration de 0,2% a 1,0% et ledit composé de catalyse contien chlorure de chaux en concentration entre 10 et 100 mM.

8. Méthode d'analyse, de séparation et de microencapsulation d'éléments cellulaires, subcellulaires ou multicellulaires, selon la revendication 7, dans lequel ladite concentration d'alginate de sodium est de 0,5% et ladite oncentration du chlorure de chaux est de 50 mM.

9. Méthode d'analyse, de séparation et de microencapsulation d'éléments cellulaires, subcellulaires ou multicellulaires, selon une quelconque des revendications précédentes, dans lequel lesdits élémentes sont d'origine végétale.

10. Méthode d'analyse, de séparation et de microencapsulation d'éléments cellulaires, subcellulaires ou multicellulaires, selon la revendication 9, dans lequel lesdits éléments végétals sont des cellules isolées d'une des espèces suivantes: Nicotiana benthamiana, Solanum tuberosum, Citrus sinensis ou leurs sub-cellules, y compris les chromosomes.
